# FASCICULE DE BREVET EUROPEEN

(11) **EP 2 102 774 B1**
(45) Date de publication et mention de la délivrance du brevet: **31.03.2010**
(21) Numéro de dépôt: 07871975.4
(22) Date de dépôt: 19.12.2007
(51) Int. Cl.: G06F 19/00

(54) **METHODE CORRECTRICE DE TRAITEMENT DE RESULTATS D'EXPERIENCES TRANSCRIPTOMIQUES OBTENUS PAR ANALYSE DIFFERENTIELLE**
KORREKTURVERFAHREN ZUR VERARBEITUNG VON DURCH DIFFERENTIELLE ANALYSE GEWONNENEN ERGEBNISSEN TRANSKRIPTOMISCHER EXPERIMENTE
CORRECTIVE METHOD FOR PROCESSING THE RESULTS OF TRANSCRIPTOMIC EXPERIMENTS OBTAINED BY DIFFERENTIAL ANALYSIS

(30) Priorité: 19.12.2006 FR 0655660
(43) Date de publication de la demande: 23.09.2009
(73) Titulaire: Galderma Research & Development, 06410 Biot (FR)
(72) Inventeur: FOGEL, Paul, F-75005 Paris (FR); ZUGAJ, Didier, F-06250 Mougins le Haut (FR); LE GOFF, Jean-Marc, F-13009 Marseille (FR); AUBERT, Jérôme, F-06130 Grasse (FR); DERET, Sophie, F-06250 Mougins (FR)
(74) Mandataire: Allab, Myriam
(86) Numéro de dépôt international: PCT/FR2007/052562
(87) Numéro de publication internationale: WO 2008/087324

(56) Documents cités:
- MANSOURIAN ROBERT ET AL: "The Global Error Assessment (GEA) model for the selection of differentially expressed genes in microarray data" BIOINFORMATICS (OXFORD), vol. 20, no. 16, 1 novembre 2004 (2004-11-01), pages 2726-2737, XP002436018 ISSN: 1367-4803
- YANG YEE HWA ET AL: "Identifying differentially expressed genes from microarray experiments via statistic synthesis" BIOINFORMATICS (OXFORD), vol. 21, no. 7, avril 2005 (2005-04), pages 1084-1093, XP002436019 ISSN: 1367-4803
- WANG SONG ET AL: "A generalized likelihood ratio test to identify differentially expressed genes from microarray data." BIOINFORMATICS (OXFORD), vol. 20, no. 1, 1 janvier 2004 (2004-01-01), pages 100-104, XP002436020 ISSN: 1367-4803

## Description

La présente invention concerne des méthodes correctrices de traitement de résultats d'expériences transcriptomiques obtenus par analyse différentielle. Elle concerne plus particulièrement le traitement de tels résultats, dans le cas d'expériences menées sur des puces à ADN. Les expériences transcriptomiques ont pour objet l'identification de gènes d'intérêt ou de groupes de gènes d'intérêt.

Généralement, le niveau d'expression de ces gènes d'intérêt ou de ces groupes de gènes d'intérêt varie de façon significative, par exemple, en réponse à un signal. Lors de l'analyse de résultats d'expériences transcriptomiques, par exemple au moyen de puces à ADN, il est courant de sélectionner les gènes présentant la plus grande modulation, c'est-à-dire la plus grande variation de leur niveau d'expression. Le niveau de cette modulation, encore appelé coefficient de modulation, se définit comme le ratio du niveau d'expression observé dans une expérience, par exemple dans une condition dite de traitement, sur celui observé dans une autre expérience, par exemple dans une condition dite de référence.

L'examen des résultats montre que, plus on utilise un niveau de modulation élevé pour restreindre le nombre de gènes sélectionnés, plus on favorise, dans la sélection réalisée, l'émergence de gènes dont le niveau d'expression est, dans la condition de référence, proche de la limite de détection. Or, il n'existe aucun argument biologique pour expliquer la raison pour laquelle les gènes les plus faiblement exprimés dans la condition de référence seraient les gènes les plus fortement modulés lors d'un traitement. Cette sélection introduit donc un biais et conduit à ignorer des gènes qui présentent un niveau de modulation moins élevé du simple fait qu'ils sont plus exprimés dans la condition de référence.

Si l'on estime le coefficient de modulation du niveau d'expression à partir de plusieurs observations d'un même gène sur plusieurs puces correspondant à la même condition, c'est-à-dire à partir de réplicats de la condition de référence ou de la condition de traitement, on démontre que le coefficient de modulation et le niveau moyen d'expression des gènes évoluent en sens inverse [R. Mansourian et *al*., The Global error assessment (GEA) model for the selection of differentially expressed genes in microarray data, Bioinformatics Advance Access, 2004]. En d'autres termes, plus le niveau d'expression d'un gène est faible dans plusieurs réplicats d'une condition de référence, plus son coefficient de modulation en réponse au traitement, calculé à partir de plusieurs réplicats, est élevé. Ce phénomène s'explique en partie par la présence d'un bruit de fond de mesure qui s'avère d'autant plus prépondérant dans le calcul du coefficient de modulation que les gènes sont faiblement exprimés.

L'analyse différentielle selon la méthode dite de Global Error Assessment (GEA) divulguée dans le document référencé ci-dessus permet de corriger ce biais. Elle consiste à regrouper les gènes selon un critère statistique, appelé significativité (ou p-value, en langue anglaise), prenant en compte la variabilité du coefficient de modulation en fonction du niveau d'expression dans la condition de référence pour chaque gène. La variabilité du coefficient de modulation représente, pour un gène donné, l'écart type des coefficients de modulation avec le coefficient de modulation moyen. Cette p-value traduit la significativité d'une valeur de coefficient de modulation. Ceci permet d'obtenir des groupes de gènes correspondant à une p-value donnée et d'équilibrer, dans la liste des gènes sélectionnés, la proportion des gènes faiblement exprimés dans la condition de référence.

Toutefois, la p-value n'a pas de sens biologique. De ce fait, le biologiste, qui raisonne dans l'univers des modulations, ne peut pas s'appuyer sur cette valeur pour identifier les gènes différenciés. En conséquence, il ne peut pas utiliser la méthode dite de GEA pour trouver les gènes différenciés.

En pratique, après une ou plusieurs analyses différentielles, le biologiste utilise le plus souvent des techniques de classification et de visualisation afin d'identifier des gènes présentant des profils de modulation d'expression similaires entre plusieurs conditions. Il s'agit par exemple de la technique de classification hiérarchique ou la classification par Décomposition Robuste en Valeurs Singulières divulguée dans le document L. Liu et *al*., Robust singular value decomposition analysis of microarray data, PNAS, 2003.

Toutefois, dans ces techniques, le biologiste est amené, du fait de limitations liées à l'affichage, ou pour se concentrer sur des analyses plus complexes telles que des analyses d'ontologie ou relatives à des sentiers métaboliques, à limiter la taille des listes de gènes sélectionnés. Aussi, il s'appuie sur les niveaux de modulation d'expression mesurés dans chaque condition, et ne prend donc pas en compte la significativité associée. L'information relative à cette significativité est ainsi perdue lors de la visualisation des niveaux de modulation après classification. En d'autres termes, le biologiste considère simplement le rapport du niveau d'expression de gènes entre deux conditions, classés par ordre décroissant selon leur coefficient de modulation. C'est la modulation standard.

Souhaitant généralement visualiser les gènes les plus modulés dans la condition de traitement, le biologiste applique alors un tri décroissant selon le niveau de modulation et ne conserve que les premiers gènes. Ce faisant, il ne tient pas compte de la significativité et réintroduit le biais de sélection qui avait été éliminé par le calcul de la p-value.

Compte tenu de ce qui précède, un problème que se propose de résoudre l'invention est de réaliser une méthode correctrice de traitement de résultats d'expériences transcriptomiques obtenus par analyse différentielle, qui prend en compte la significativité associée aux valeurs de coefficient de modulation, et dont le résultat est en outre exploitable à partir de valeurs ayant un sens biologique.

La solution proposée de l'invention à ce problème a pour objet une méthode correctrice de traitement de résultats d'expériences transcriptomiques obtenus par analyse différentielle, comprenant les étapes suivantes :
- obtention des résultats du niveau d'expression de gènes dans une condition de référence, et calcul du niveau moyen d'expression de chacun desdits gènes;
- obtention des résultats du niveau d'expression desdits gènes dans une condition de traitement, et calcul du niveau moyen d'expression pour chacun desdits gènes;
- calcul du coefficient de modulation du niveau d'expression pour chacun desdits gènes;
- calcul d'une p-value associée à chaque coefficient de modulation; et
- calcul de courbes isobares de p-value en fonction du niveau moyen d'expression de chacun desdits gènes dans la condition de référence ;
**caractérisée en ce qu**'elle comprend en outre une étape de calcul et d'association d'un coefficient de modulation médian sur la courbe isobare de chaque p-value observée.

De manière avantageuse, lesdites étapes de calcul d'une p-value associée à chaque coefficient de modulation et de calcul d'un coefficient de modulation médian suivant la courbe isobare de chaque p-value observée sont réalisées au moyen de la méthode dite GEA ; lesdites étapes d'obtention des résultats du niveau d'expression de gènes dans une (des) condition(s) de référence, d'obtention des résultats du niveau d'expression de gènes dans une (des) condition(s) de traitement, de calcul du coefficient de modulation (variation) du niveau d'expression, calcul de la p-value, et de calcul d'un coefficient de modulation médian sur la courbe isobare de chaque p-value observée sont menées pour une pluralité de conditions de traitement différentes ; ladite étape de calcul de courbes isobares de p-value en fonction du niveau moyen d'expression de chacun desdits gènes dans la condition de référence comprend une représentation par un point de chaque gène étudié, sur un graphe présentant, en abscisse, le logarithme du niveau moyen d'expression dans la condition de référence noté *x* et, en ordonnée, le logarithme du niveau moyen d'expression dans la condition de traitement noté *y*, une courbe isobare de niveau *p* correspondant aux points théoriques pour lesquels la p-value est égale à p; un utilisateur sélectionne des gènes d'intérêt sur la base d'une valeur ayant un sens biologique ; ladite valeur ayant un sens biologique est une valeur de coefficient de modulation ; un utilisateur sélectionne des gènes d'intérêt sur la base d'une valeur ayant une significativité ; les expériences transcriptomiques sont menées sur des puces à ADN.

L'invention concerne en outre un ordinateur pour la mise en oeuvre d'une méthode correctrice de traitement de résultats d'expériences transcriptomiques obtenus par analyse différentielle selon l'invention.

L'invention sera mieux comprise à la lecture de la description non limitative qui suit et des dessins qui l'accompagnent, dans lesquels :

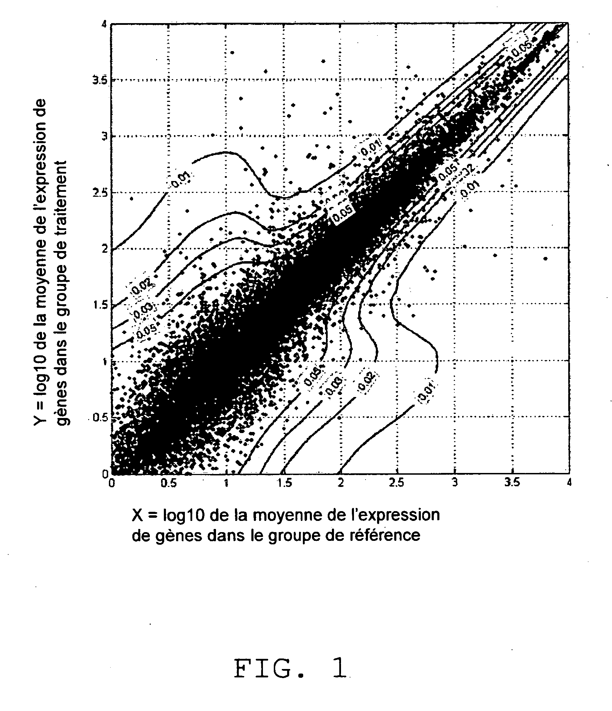

- la figure 1 représente sous forme de graphe les résultats d'expériences transcriptomiques, ainsi que les courbes isobares de p-value, telles qu'elles peuvent être obtenues selon un mode de réalisation de la présente invention ;

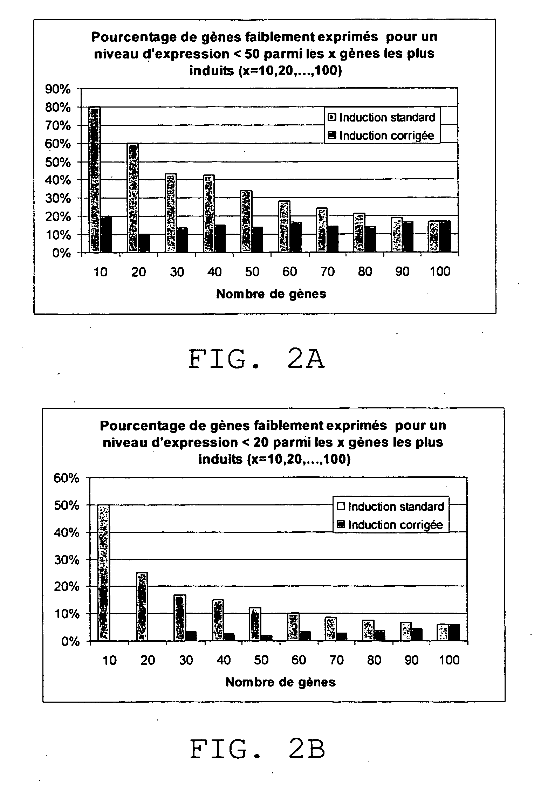

- les figures 2A et 2B représentent sous forme d'histogrammes la répartition des gènes faiblement exprimés, c'est-à-dire dont l'expression est inférieure à 50 (2A) ou inférieure à 20 (2B)(unité arbitraire liée à la technologie Affymetrix^{™}), parmi les gènes les plus modulés, résultant de l'analyse d'expériences transcriptomiques, d'une part, selon la méthode de modulation standard et, d'autre part, selon l'invention, dite méthode de modulation corrigée.

La méthode de traitement selon l'invention, est une méthode correctrice qui permet le traitement de résultats d'expériences transcriptomiques et, en particulier, le traitement de résultats d'expériences transcriptomiques menées sur des puces à ADN.

Les expériences transcriptomiques menées sur des puces à ADN sont susceptibles d'être mises en oeuvre de la manière suivante.

Des cellules sont cultivées dans au moins deux conditions différentes. La première condition est dite de référence. Elle sert de contrôle. La seconde condition est dite de traitement. Dans la condition de traitement, les cellules sont cultivées soit en présence d'un agent particulier, par exemple une protéine ou un antibiotique, soit dans des conditions expérimentales particulières, par exemple, de luminosité, d'oxygénation, de pH ou de pression.

En pratique, chaque culture est avantageusement réalisée plusieurs fois pour une même condition. On a alors une pluralité de réplicats. Par pluralité de réplicats on entend de préférence trois ou quatre cultures réalisées dans une condition de référence, et deux cultures réalisées dans une même condition de traitement. Les résultats obtenus à partir de plusieurs réplicats permettent une analyse statistique.

De façon indépendante pour chaque réplicat, les cellules sont lysées et les acides nucléiques de ces cellules sont solubilisés. Les ARNm sont ensuite purifiés par passage sur colonne contenant des billes où sont fixés des oligomères oligo-dT. En effet, les ARNm, qui possèdent une queue polyadénylée, sont retenus par les billes de la colonne alors que les autres acides nucléiques sont éliminés.

Après élution, les ARNm purifiés pour chaque réplicat sont récupérés. Des ADNc fluorescents sont alors synthétisés à partir des ARNm obtenus. Pour ce faire, des amorces soit oligo-dT, soit spécifiques de chaque ARNm, soit aléatoires, s'hybrident avec les ARNm purifiés et une enzyme, la reverse transcriptase, permet la synthèse du brin d'ADNc. L'utilisation de nucléotides fluorescents permet d'obtenir des ADNc marqués. Les ARNm sont dégradés pour ne conserver plus que les simples brins d'ADNc fluorescents.

Les niveaux d'expression des gènes pour chaque réplicat sont mesurés au moyen d'une puce à ADN. Une telle puce comporte classiquement plusieurs milliers de puits. Plusieurs milliers d'exemplaires d'une même séquence codante sont fixés dans chaque puits et cette séquence codante est différente d'un puits à l'autre.

La puce à ADN est mise en présence de la solution comportant les ADNc fluorescents. Ces ADNc s'hybrident spécifiquement sur la séquence qui leur est complémentaire.

Après une ou plusieurs étapes de lavage, la puce est placée dans un instrument de détection et scannée à l'aide d'un laser qui excite les fluorochromes.

Pour chaque puits, on détermine alors une intensité de fluorescence. Cette intensité est proportionnelle à la quantité d'ADNc fluorescent fixée. Chaque intensité est proportionnelle au niveau d'expression du gène considéré. Par conséquent, à partir des intensités de fluorescence, on obtient pour chaque gène analysé et pour chaque expérience, une valeur de niveau d'expression.

La présente invention concerne une méthode correctrice de traitement de résultats d'expériences transcriptomiques obtenus par analyse différentielle des valeurs d'expression de gènes, dont le but est de sélectionner les gènes les plus modulés, c'est-à-dire les plus fortement sur-exprimés, et/ou les plus fortement inhibés, dans la ou les conditions de traitement par rapport à la condition de référence.

En particulier, la méthode selon la présente invention comprend une étape de calcul du niveau d'expression moyen de chaque gène analysé, à partir de l'ensemble des réplicats d'une même condition. Ladite méthode comprend ensuite, pour chaque gène, le calcul d'un coefficient de modulation d'expression, c'est-à-dire du ratio entre son niveau d'expression moyen dans la condition traitée et dans la condition de référence.

Dans une autre étape de la méthode selon l'invention, pour chaque gène analysé, une p-value est calculée, associée au coefficient de modulation dudit gène. Cette valeur statistique traduit la chance d'observer une différence, au moins d'un certain niveau, entre les niveaux d'expression d'un gène dans deux conditions différentes, alors, qu'en réalité, ces niveaux d'expression sont identiques. En d'autres termes, cette p-value traduit la mesure dans laquelle la valeur d'un coefficient de modulation est significative, ou est au contraire due à un bruit de fond. Ainsi, cette p-value prend en compte la variabilité du coefficient de modulation du niveau d'expression dudit gène. Différentes méthodes, comme par exemple la méthode dite de GEA, sont applicables à la présente méthode pour déterminer cette p-value.

Une autre étape de la méthode de traitement selon l'invention comprend le calcul de courbes isobares de la p-value en fonction du niveau moyen d'expression dans la condition de référence. Cette étape peut être représentée graphiquement, ainsi que cela est montré à la figure 1. Pour cela, on représente par un point chaque gène étudié, sur un graphe présentant, en abscisse, le logarithme du niveau moyen d'expression dans la condition de référence noté *x* et, en ordonnée, le logarithme du niveau moyen d'expression dans la condition de traitement noté *y*. Une courbe isobare de niveau *p* correspond aux points *théoriques* pour lesquels la p-value est égale à p. Le processus de construction des courbes isobares est le suivant :
1) On construit un quadrillage suffisamment fin du plan *xy* délimité par les valeurs extrêmes d'expression observées ;
2) On détermine pour chacun des points du quadrillage la différence *y-x* entre la condition de traitement et la condition de référence, que l'on divise par l'écart-type associé au niveau d'expression moyen (*x+y*)/2. A ces fins, on aura préalablement établi la relation existant entre niveau d'expression et variance d'un gène sur la base des réplicats de la condition de référence, conformément à la méthode de GEA ;
3) On trace par une méthode usuelle d'obtention de contours (par exemple référence Matlab) les courbes isobares correspondant aux triplicats (*x*,*y*,p-value(*x*,*y*)). Pour une même p-value, les gènes surexprimés sont rassemblés sur une courbe, tandis que les gènes réprimés sont rassemblés sur une autre courbe.

La dernière étape de la méthode consiste, pour chaque courbe isobare de la p-value, à lui associer la modulation médiane des points contenus dans cette courbe. A ces fins, on considère l'ensemble des points du quadrillage en abscisse, auxquels on associe en ordonnée les points correspondants sur la courbe isobare. A chacun des points (*x*,*y*) ainsi obtenus correspond une modulation exp(*y*-*x*). Dans le calcul de la médiane proprement dit, chaque point (*x*,*y*) est pondéré par la densité de gènes observée dans la condition de référence autour de la valeur x.

La modulation corrigée étant par construction une fonction monotone de la p-value, elle permet d'obtenir les mêmes tris de gènes que les méthodes selon l'état de la technique, et avec, de manière avantageuse, des niveaux de modulation corrigée mieux distribués. En particulier, les tris ne comprennent pas de valeurs aberrantes consécutives à des expressions atteignant, dans une des conditions, un niveau très faible, donc peu fiable car inférieur au seuil de détection des puces à ADN. Ainsi, la méthode selon l'invention permet de mieux distribuer les gènes faiblement exprimés dans la condition de référence parmi les gènes les plus modulés.

De manière avantageuse, les résultats sont analysés dans l'univers des niveaux de modulation, et les sélections, ou tris de listes, sont effectués dans le même univers et non pas l'univers des p-value. En particulier, la méthode selon l'invention tient compte de la variabilité des coefficients de modulation en fonction du niveau d'expression pour chaque gène, de la significativité associée et du niveau de modulation médian, pour identifier des profils de modulation similaire, tout en ne perdant aucune information significative.

De manière encore avantageuse, la méthode selon l'invention permet au biologiste de raisonner dans un seul et même espace, de conserver ses habitudes et de vaincre le préjugé selon lequel il n'est pas possible de travailler en tenant compte de la p-value tout en gardant un sens biologique aux résultats. En effet, le biologiste peut désormais choisir une valeur biologique, et non plus une valeur statistique comme seuil de sélection des gènes d'intérêt. En effet, il peut désormais sélectionner les gènes d'intérêt suffisamment modulés, selon son jugement, directement à partir d'une valeur seuil de coefficient de modulation ayant un sens biologique et une significativité associée. La méthode selon l'invention ayant regroupés les gènes en sous-ensembles, à la fois en fonction de la valeur médiane de leur coefficient de modulation, et de la significativité de ces coefficients de modulation, le biologiste sélectionne alors des gènes en fonction de la significativité de leur coefficient de modulation.

Les figures 2A et 2B représentent des analyses différentielles de résultats d'expériences transcriptomiques obtenus à partir d'une condition de traitement par rapport à une condition de référence (culture de sébocytes préputiaux de rat en présence (condition de traitement) ou non (condition de référence) d'un agoniste de PPARgamma, le composé CD4700)).

Dans la figure 2A, on sélectionne les gènes les plus fortement modulés et on représente sous forme d'histogrammes les pourcentages de gènes ayant une intensité d'expression inférieure à 50 (unité arbitraire liée à la technologie Affymetrix^{™}), parmi les premiers gènes sélectionnés. Ces sélectons sont effectuées d'une part avec la méthode de modulation corrigée selon l'invention, et d'autre part avec la méthode standard de l'état de la technique. En comparant ces deux méthodes, on constate que la méthode selon l'invention uniformise la distribution de ces gènes faiblement exprimés. En d'autres termes, en appliquant la méthode de modulation corrigée selon l'invention, on obtient environ 15% de gènes ayant une intensité d'expression inférieure à 50 lors de la sélection des 10 gènes les plus modulés ou des 100 gènes les plus modulés. Au contraire, la méthode précitée de l'état de la technique ne donne pas de résultats uniformes. Par exemple, avec la méthode standard, parmi les 10 gènes les plus modulés on retrouve 80% de gènes ayant une intensité d'expression inférieure à 50.

Dans la figure 2B, on sélectionne les gènes les plus fortement modulés et on représente sous forme d'histogrammes les pourcentages de gènes ayant une intensité d'expression inférieure à 20 (unité arbitraire liée à la technologie Affymetrix^{™}), parmi les premiers gènes sélectionnés, selon la méthode de modulation corrigée et selon la méthode de modulation standard. De manière similaire à la figure 2A, on obtient une uniformisation de la distribution des gènes ayant une intensité d'expression inférieure à 20 parmi les gènes les plus modulés sélectionnés avec la méthode de modulation corrigée selon l'invention.

Dans un autre exemple, non représenté on a mené une analyse statistique différentielle de l'expression de gènes au moyen d'une puce à ADN dont la référence est RAE230A, ces gènes étant modulés par un agoniste PPAR . Deux méthodologies ont été suivies afin d'étudier les gènes, correspondant aux identifiants Affymetrix^{™} contenus dans les puits de la puce, en fonction du niveau d'expression des gènes : la méthodologie dite standard, correspondant à la méthode dite GEA et la méthodologie dite corrigée de l'invention. Les deux listes obtenues ont été triées en fonction du niveau d'expression des gènes, par ordre décroissant. Les 50 premiers identifiants Affymetrix^{™} de chaque liste ont été analysés avec l'application web GOTM^{™} décrite dans la publication suivante Zhang B, Schmoyer D, Kirov S, Snoddy J. (2004), BMC Bioinformatics, 18;5(1):16.

Si l'on s'intéresse à une ontologie relative au « métabolisme des lipides », qui constitue l'une des ontologies les plus significativement touchées sur le plan statistique, il apparaît que, avec la méthode standard, 7 identifiants Affymetrix^{™} correspondant à 5 gènes sont extraits alors que, avec la méthode selon l'invention, 10 identifiants Affymetrix^{™} correspondant à 7 gènes ont été identifiés.

Il est bien entendu que la méthode correctrice selon l'invention peut, en pratique, être mise en oeuvre par un ordinateur du type personnel muni au moins d'un microprocesseur et de mémoires ROM et RAM associées. Il constitue alors un logiciel dont l'exécution est contrôlée par un biologiste ou un autre opérateur, pour l'obtention de résultats corrigés d'expériences transcriptomiques. Ce logiciel peut par ailleurs être enregistré sur tous supports mémoire du type non-volatile ou non tels que des CD-ROM ou disquettes.

## Revendications

1. Méthode correctrice mise en oeuvre par ordinateur de traitement de résultats d'expériences transcriptomiques obtenus par analyse différentielle, comprenant les étapes suivantes :
- obtention des résultats du niveau d'expression de gènes dans une condition de référence, et calcul du niveau moyen d'expression de chacun desdits gènes,
- obtention des résultats du niveau d'expression desdits gènes dans une condition de traitement, et calcul du niveau moyen d'expression pour chacun desdits gènes,
- calcul du coefficient de modulation du niveau d'expression pour chacun desdits gènes,
- calcul d'une p-value associée à chaque coefficient de modulation,
- calcul de courbes isobares de p-value en fonction du niveau moyen d'expression de chacun desdits gènes dans la condition de référence ;
**caractérisée en ce qu'**elle comprend en outre une étape de calcul et d'association d'un coefficient de modulation médian sur la courbe isobare de chaque p-value observée.

2. Méthode selon la revendication 1, **caractérisée en ce que** lesdites étapes de calcul d'une p-value associée à chaque coefficient de modulation et de calcul d'un coefficient de modulation médian suivant la courbe isobare de chaque p-value observée sont réalisées au moyen de la méthode dite GEA.

3. Méthode selon l'une des revendications 1 ou 2, **caractérisée en ce que** lesdites étapes d'obtention des résultats du niveau d'expression de gènes dans une condition de référence, d'obtention des résultats du niveau d'expression de gènes dans une condition de traitement, de calcul du coefficient de modulation du niveau d'expression, calcul de la p-value , et de calcul d'un coefficient de modulation médian sur la courbe isobare de chaque p-value observée sont menées pour une pluralité de conditions de traitement différentes.

4. Méthode selon l'une des revendications 1, 2 ou 3, **caractérisée en ce que** ladite étape de calcul de courbes isobares de p-value en fonction du niveau moyen d'expression de chacun desdits gènes dans la condition de référence comprend une représentation par un point de chaque gène étudié, sur un graphe présentant, en abscisse, le logarithme du niveau moyen d'expression dans la condition de référence noté *x* et, en ordonnée, le logarithme du niveau moyen d'expression dans la condition de traitement noté *y*, une courbe isobare de niveau *p* correspondant aux points théoriques pour lesquels la p-value est égale à p.

5. Méthode selon l'une des revendications 1 à 4, **caractérisée en ce qu'**un utilisateur sélectionne des gènes d'intérêt sur la base d'une valeur ayant un sens biologique.

6. Méthode selon la revendication 5, **caractérisée en ce** ladite valeur ayant un sens biologique est une valeur de coefficient de modulation.

7. Méthode selon l'une des revendications précédentes, **caractérisée en ce qu'**un utilisateur sélectionne des gènes d'intérêt sur la base d'une valeur ayant une significativité.

8. Méthode selon l'une des revendications précédentes, **caractérisée en ce que** les expériences transcriptomiques sont menées sur des puces à ADN.

9. Ordinateur pour la mise en oeuvre d'une méthode correctrice de traitement de résultats d'expériences transcriptomiques obtenus par analyse différentielle selon l'une des revendications précédentes.

## Claims

1. Computer-implemented corrective method for processing the results of transcriptome experiments obtained by differential analysis, comprising the following steps:
- obtaining results of the gene expression level under a reference condition and calculating the average expression level of each of said genes,
- obtaining results of the expression level of said genes under a processing condition and calculating the average expression level for each of said genes,
- calculating the modulation coefficient for the expression level for each of said genes,
- calculating a p-value associated with each modulation coefficient,
- calculating isobaric curves of p-value as a function of the average expression level of each of said genes under the reference condition;
**characterized in that** it also comprises a step of calculating and associating a median modulation coefficient on the isobaric curve of each p-value observed.

2. Method according to Claim 1, **characterized in that** said steps of calculating a p-value associated with each modulation coefficient and of calculating a median modulation coefficient according to the isobaric curve of each p-value observed are carried out by means of the method termed GEA.

3. Method according to either of Claims 1 and 2, **characterized in that** said steps of obtaining results of the gene expression level under a reference condition, of obtaining results of the gene expression level under a processing condition, of calculating the modulation coefficient for the expression level, of calculating the p-value and of calculating a median modulation coefficient on the isobaric curve of each p-value observed are carried out for a plurality of different processing conditions.

4. Method according to one of Claims 1, 2 or 3, **characterized in that** said step of calculating isobaric curves of p-value as a function of the average expression level of each of said genes under the reference condition comprises a representation, by a dot, of each gene studied, on a graph displaying, on the x-axis, the logarithm of the average expression level under the reference condition noted *x* and, on the y-axis, the logarithm of the average expression level under the processing condition noted *y*, an isobaric curve of level *p* corresponding to the theoretical dots for which the p-value is equal to p.

5. Method according to one of Claims 1 to 4, **characterized in that** a user selects genes of interest on the basis of a value which has a biological meaning.

6. Method according to Claim 5, **characterized in that** said value which has a biological meaning is a modulation coefficient value.

7. Method according to one of the preceding claims, **characterized in that** a user selects genes of interest on the basis of a value which has a significance.

8. Method according to one of the preceding claims, **characterized in that** the transcriptome experiments are carried out on DNA chips.

9. Computer for implementing a corrective method for processing the results of transcriptome experiments obtained by differential analysis according to one of the preceding claims.

## Patentansprüche

1. Mit einem Computer durchgeführtes Korrekturverfahren zur Verarbeitung der Ergebnisse von Transkriptomik-Experimenten, die durch differentielle Analyse gewonnen wurden, das die folgenden Schritte umfasst:
- Gewinnung von Ergebnissen bezüglich des Niveaus der Genexpression unter einer Referenzbedingung und Berechnung des mittleren Expressionsniveaus für jedes Gen,
- Gewinnung von Ergebnissen bezüglich des Expressionsniveaus dieser Gene unter einer Behandlungsbedingung und Berechnung des mittleren Expressionsniveaus für jedes Gen,
- Berechnung des Änderungskoeffizienten des Expressionsniveaus für jedes Gen,
- Berechnung eines p-Werts, der mit jedem Änderungskoeffizienten verknüpft ist,
- Berechung der isobaren Kurven des p-Werts in Abhängigkeit vom mittleren Expressionsniveau jedes dieser Gene unter der Referenzbedingung,
**dadurch gekennzeichnet, dass** es ferner einen Schritt der Berechung und Zuordnung eines mittleren Änderungskoeffizienten zu der isobaren Kurve jedes erhaltenen p-Werts umfasst.

2. Verfahren nach Anspruch 1, **dadurch gekennzeichnet, dass** die Schritte der Berechung eines mit jedem Änderungskoeffizienten verknüpften p-Wertes und der Berechnung eines mittleren Änderungskoeffizienten gemäß der isobaren Kurve jedes erhaltenen p-Werts mit Hilfe der so genannten GEA-Methode durchgeführt wird.

3. Verfahren nach einem der Ansprüche 1 oder 2, **dadurch gekennzeichnet, dass** die Schritte der Gewinnung von Ergebnissen bezüglich des Niveaus der Genexpression unter einer Referenzbedingung, der Gewinnung von Ergebnissen bezüglich des Niveaus der Genexpression unter einer Behandlungsbedingung, der Berechnung des Änderungskoeffizienten des Expressionsniveaus, der Berechnung des p-Werts und der Berechnung eines mittleren Änderungskoeffizienten gemäß der isobaren Kurve jedes erhaltenen p-Werts für eine Vielzahl von unterschiedlichen Behandlungsbedingungen durchgeführt werden.

4. Verfahren nach einem der Ansprüche 1, 2 oder 3, **dadurch gekennzeichnet, dass** der Schritt der Berechung der isobaren Kurven des p-Werts in Abhängigkeit vom mittleren Expressionsniveau jedes Gens unter der Referenzbedingung eine Darstellung jedes untersuchten Gens durch einen Punkt in einer grafischen Darstellung umfasst, bei der an der x-Achse der als x bezeichnete Logarithmus des mittleren Expressionsniveaus unter der Referenzbedingung und an der y-Achse der als y bezeichnete Logarithmus des mittleren Expressionsniveaus unter der Behandlungsbedingung aufgetragen wird, wobei eine isobare Kurve des Niveaus p den theoretischen Punkten entspricht, für die der p-Wert p beträgt.

5. Verfahren nach einem der Ansprüche 1 bis 4, **dadurch gekennzeichnet, dass** der Anwender die interessierenden Gene auf der Basis eines Wertes mit biologischer Bedeutung auswählt.

6. Verfahren nach Anspruch 5, **dadurch gekennzeichnet, dass** der Wert mit biologischer Bedeutung ein Wert des Änderungskoeffizienten ist.

7. Verfahren nach einem der vorhergehenden Ansprüche, **dadurch gekennzeichnet, dass** der Anwender die interessierenden Gene auf der Basis eines Wertes mit Signifikanz auswählt.

8. Verfahren nach einem der vorhergehenden Ansprüche, **dadurch gekennzeichnet, dass** die Transkriptomik-Experimente an DNA-Chips durchgeführt werden.

9. Computer für die Durchführung eines Korrekturverfahrens zur Verarbeitung der Ergebnisse von Transkriptomik-Experimenten, die durch differentielle Analyse gewonnen wurden, nach einem der vorhergehenden Ansprüche.
